# FASCICULE DE BREVET EUROPEEN

(11) **EP 3 302 646 B1**
(45) Date de publication et mention de la délivrance du brevet: **04.08.2021**
(21) Numéro de dépôt: 16741052.1
(22) Date de dépôt: 02.06.2016
(51) Int. Cl.: A61M 5/20, A61M 5/315, A61M 5/32

(54) **AUTOINJECTEUR AVEC UN DISPOSITIF RETARDATEUR COMPORTANT UN TRAIN EPICYCLOIDAL**
AUTOINJEKTOR MIT EINER VERZÖGERUNGSVORRICHTUNG MIT EINEM PLANETENGETRIEBE
AUTO-INJECTOR WITH A DELAY DEVICE COMPRISING AN EPICYCLIC GEAR TRAIN

(30) Priorité: 05.06.2015 FR 1555156
(43) Date de publication de la demande: 11.04.2018
(73) Titulaire: Aptar France SAS, 27110 Le Neubourg (FR)
(72) Inventeur: FABIEN, David, 29810 Plouarzel (FR); HIS, Olivier, 27430 Saint Etienne Du Vauvray (FR); GOMEZ, Thomas, 33160 Saint Aubin De Medoc (FR); SAUSSAYE, Anthony, 61300 Saint Sulpice Sur Risle (FR)
(74) Mandataire: CAPRI
(86) Numéro de dépôt international: PCT/FR2016/051318
(87) Numéro de publication internationale: WO 2016/193627

(56) Documents cités:
- WO-A1-2013/078200
- WO-A1-2013/175139
- WO-A1-2013/175140
- WO-A1-2013/175144

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois un certain nombre d'inconvénients.

Ainsi, notamment lorsque le volume de produit fluide est relativement important et/ou lorsque le produit fluide injecté est relativement visqueux, il est souhaitable de permettre au produit de diffuser du site d'injection pendant quelques secondes après ladite injection. Si l'utilisateur retire l'autoinjecteur immédiatement après la fin de l'injection, une partie du produit peut ressortir du corps de l'utilisateur ce qui diminue l'efficacité du traitement. Il est donc souhaitable de prévoir que l'utilisateur maintienne l'autoinjecteur contre son corps encore pendant quelques secondes après la fin de l'injection. Cet aspect est généralement résolu par les autoinjecteurs existants par la notice d'utilisation qui demande à l'utilisateur de compter dans sa tête un certain nombre de secondes avant de retirer le dispositif. Ceci n'est pas fiable et donc insatisfaisant, car le système dépend alors de l'utilisateur lui-même, qui dans certains cas peut être perturbé ou affaibli par l'action d'injection qu'il vient de réaliser.

Les documents WO2013175139, WO2013175140, WO2013175144 et WO2013078200 décrivent des autoinjecteurs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable d'utilisation, qui permet à l'utilisateur de déterminer quand il doit retirer ou quand il peut retirer l'autoinjecteur de son corps après son utilisation, qui soit sûr et qui empêche tout risque de blessure, et qui soit simple et peu coûteux à fabriquer et à assembler.

La présente invention a donc pour objet un autoinjecteur tel que décrit dans la revendication 1. Des modes de réalisation avantageux sont décrits dans les revendications dépendantes. Ces caractéristiques et avantages et d'autres de la présente invention apparaîtront plus clairement au cours de la description détaillée suivante, faite en référence aux dessins joints, donnés à titre d'exemples non limitatifs, et sur lesquels :
Les figures 1a et 1b sont des vues schématiques, respectivement de côté et en section transversale, d'un autoinjecteur selon un mode de réalisation avantageux de la présente invention, en position de repos avant piquage,
Les figures 2a et 2b sont des vues similaires à celles des figures 1a et 1b, en position après piquage et avant injection,
Les figures 3a et 3b sont des vues similaires à celles des figures 2a et 2b, en position juste avant la fin d'injection et au moment du déclenchement du système retardateur,
Les figures 4a et 4b sont des vues similaires à celles des figures 3a et 3b, en position de fin d'injection et après déclenchement du système retardateur,
Les figures 5a et 5b sont des vues similaires à celles des figures 4a et 4b, en position de fin d'actionnement du système retardateur, avant retrait de l'autoinjecteur du site d'injection,
La figure 6 est une vue similaire à celle de la figure 5a, en position de fin d'utilisation, après retrait de l'autoinjecteur du site d'injection,
La figure 7 est une vue en perspective éclatée du système retardateur,
La figure 8 est une vue en perspective de détail du corps supérieur,
La figure 9 est une vue en perspective de détail de l'élément indicateur du dispositif d'indication visuel, sonore et/ou tactile,
La figure 10 est une vue en perspective de détail de la clé de verrouillage,
La figure 11 est une vue en perspective de détail du corps intermédiaire,
Les figures 12a et 12b sont des vues schématiques, respectivement en perspective éclatée et en perspective coupée, du système retardateur selon un mode de réalisation avantageux de la présente invention, dans la position des figures 1a et 1b,
Les figures 13a et 13b sont des vues similaires à celles des figures 12a et 12b, dans la position des figures 3a et 3b,
La figures 14 est une vue similaire à celle de la figure 13a, dans la position des figures 5a et 5b,
La figure 15 est une vue schématique de détail en section transversale d'une partie de l'autoinjecteur des figures 1 à 6, montrant plus particulièrement le système retardateur, dans la position des figures 1b et 2b,
Les figures 16a à 16d sont des vues schématique en coupe transversale, respectivement selon les plans de coupe A-A, B-B, C-C et D-D de la figure 15,
La figure 17 est une vue schématique en coupe transversale selon le plan de coupe E-E de la figure 15, illustrant une variante de réalisation avantageuse,
Les figures 18a et 18b sont des vues de détails en perspective d'une autre variante de réalisation, et
La figure 19 est une vue de détail en perspective d'encore une autre variante de réalisation.

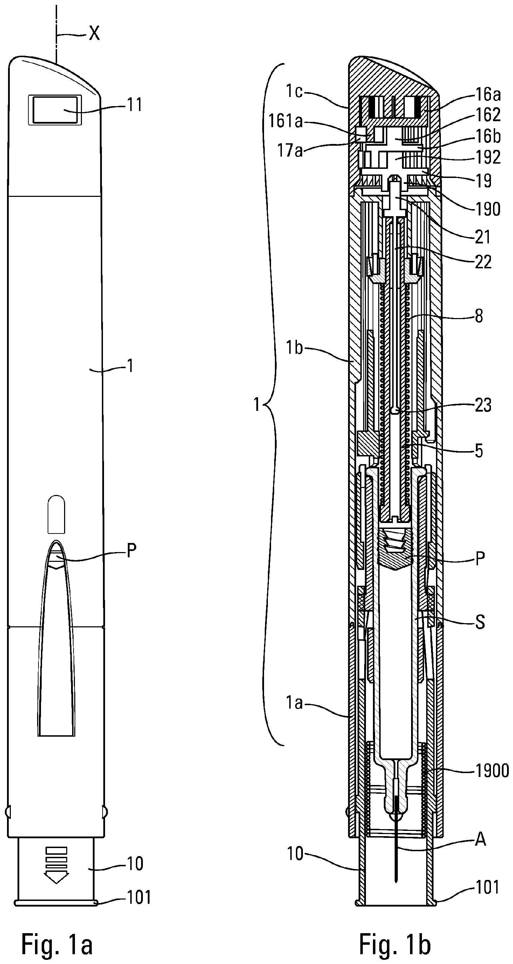

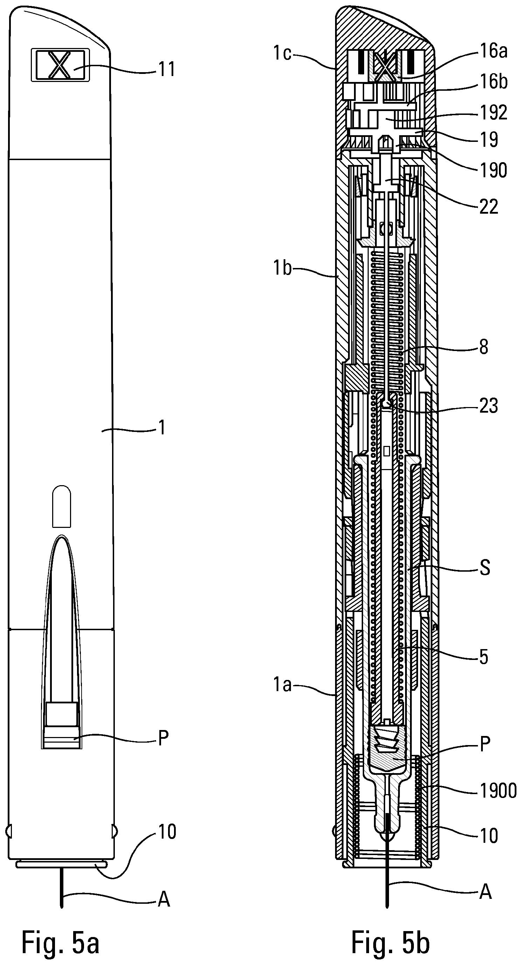

Dans la description ci-après, les termes "supérieur", "inférieur", "haut" et "bas" se réfèrent aux positions représentées sur les figures 1a à 6 et 15. Les termes "axial" et "radial" se réfèrent à l'axe central longitudinal X, représenté notamment sur la figure 1a, qui correspond à l'axe longitudinal de l'aiguille.

L'autoinjecteur va être décrit ci-après en référence à un mode de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

L'autoinjecteur représenté sur les figures comporte un corps 1 dans lequel coulisse axialement un manchon actionneur 10, dont l'extrémité inférieure 101 est destinée à venir en contact avec le corps du patient autour de la zone d'injection. Dans l'exemple représenté sur les figures, l'autoinjecteur comporte un corps inférieur 1a, un corps intermédiaire 1b et un corps supérieur 1c qui sont assemblés pour former le corps 1 de l'autoinjecteur, comme indiqué sur la figure 1b. Ci-après, et dans les autres figures, le terme "corps" et la référence numérique "1" seront utilisés pour désigner ledit corps unitaire formé par l'assemblage dudit corps inférieur 1a avec ledit corps intermédiaire 1b et ledit corps supérieur 1c. Il est à noter que le corps 1 pourrait être formé d'un nombre quelconques de parties de corps, par exemple deux, et que le mode de réalisation des figures, avec trois parties de corps, n'est pas limitatif.

Un réservoir S peut être inséré dans ledit corps 1 de l'autoinjecteur, ledit réservoir S étant fixe dans ledit corps 1. Ce réservoir S contient du produit fluide, et comporte un piston P et une aiguille A. Le piston P est adapté à se déplacer dans ledit réservoir S pour injecter le produit fluide à travers ladite aiguille A. Eventuellement, la présente invention pourrait aussi s'appliquer à un réservoir sans aiguille, notamment dans un dispositif d'injection sans aiguille.

La présente description sera faite en référence à une seringue S, qui peut être de tout type. Plus généralement, il est entendu que le terme "seringue" dans la présente description englobe tout type de réservoir associé à une aiguille. De préférence, le réservoir S est une seringue préremplie.

Avant actionnement de l'autoinjecteur, l'aiguille A de la seringue S peut être protégée par un capuchon (non représenté), l'autoinjecteur pouvant comporter un capot (non représenté) que l'utilisateur peut retirer avant actionnement. Le retrait du capot provoque avantageusement le retrait du capuchon.

Avant actionnement, le manchon actionneur 10 est dans une première position projetée dans laquelle il entoure l'aiguille A, comme représenté sur les figures 1a et 1b. Lors de l'actionnement, le manchon actionneur 10 coulisse à l'intérieur du corps 1 vers une position d'actionnement, pour exposer l'aiguille A et permettre le piquage puis l'injection du produit fluide.

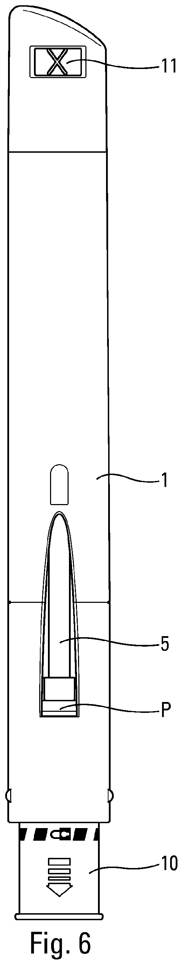

Après l'injection, lorsque l'utilisateur retire l'autoinjecteur du site d'injection, le manchon actionneur 10 revient dans une seconde position projetée de fin d'utilisation, dans laquelle il est à nouveau disposé autour de l'aiguille A, pour éviter tout risque de blessure avec ladite aiguille, comme représenté sur la figure 6.

Le manchon actionneur 10 est avantageusement sollicité vers ses positions projetées par un organe élastique ou ressort 1900, qui peut être de type quelconque. Avantageusement, dans ladite position de fin d'utilisation, ledit manchon actionneur 10 est verrouillé, et ne peut plus être déplacé axialement vers l'intérieur dudit corps 1. Ce verrouillage peut par exemple être réalisé par des pattes (non représentées), solidaires du corps 1 ou du réservoir S, et coopérant avec des ouvertures (non représentées) dudit manchon actionneur 10 lorsque celui-ci atteint sa seconde position projetée. Ce verrouillage, qui n'est pas essentiel au fonctionnement de la présente invention, ne sera pas décrit plus en détail ci-après. Il pourrait être réalisé différemment du mode de réalisation particulier mentionné ci-dessus. En particulier, il pourrait être réalisé conformément aux enseignements des documents WO2013175140 ou WO2013175142.

L'autoinjecteur comporte également un système d'injection automatique, comportant notamment une tige de piston 5 adaptée à coopérer avec le piston P pour le déplacer dans le réservoir S pour distribuer le produit fluide à travers l'aiguille A. Cette tige de piston 5 est classiquement sollicitée par un ressort d'injection 8 vers sa position de distribution, et retenue avant actionnement dans sa position de repos par une serrure d'injection appropriée.

Une serrure d'injection avantageuse est notamment décrite dans le document WO2015155484.

La serrure peut comprendre au moins un élément de blocage retenu en position de blocage par une bague de blocage. Le déclenchement de ladite serrure d'injection provoque l'actionnement desdits moyens d'injection et donc l'injection du produit fluide à travers l'aiguille. Ladite serrure d'injection peut en outre comporter un manchon de commande 4 disposé dans ledit corps 1, ledit manchon de commande 4 contenant ladite tige de piston 5 et ledit ressort d'injection 8, ladite tige de piston 5 comportant un évidement radial recevant au moins un élément de blocage mobile entre une position de blocage et une position de déblocage. Ledit au moins un élément de blocage est de préférence de forme sensiblement sphérique, tel qu'une bille. Avantageusement, lesdites billes sont sollicitées radialement vers l'extérieur par ladite tige de piston 5 et sont retenues en position de blocage par la bague de blocage. Cette bague de blocage est déplaçable axialement par rapport à ladite tige de piston 5 entre une position de verrouillage, dans laquelle elle maintient lesdites billes en position de blocage, et une position de déverrouillage, dans laquelle lesdites billes sont libérés pour ainsi débloquer ladite serrure d'injection, permettant audit ressort d'injection 8 de déplacer ladite tige de piston 5 vers sa position d'injection.

Lorsque l'aiguille A de la seringue S a pénétré le corps de l'utilisateur, la bague de blocage est déplacée axialement vers le haut, ce qui a pour effet de libérer les billes de leur position de blocage, celles-ci étant alors déplacées radialement vers l'extérieur. La tige de piston 5 n'est alors plus retenue par les billes et elle est donc déplacée axialement vers le bas pour réaliser l'injection du produit.

L'autoinjecteur comporte un dispositif d'indication visuel, sonore et/ou tactile pour indiquer à l'utilisateur, notamment par un son audible, par une vibration et/ou par une indication visuelle et/ou tactile, qu'il peut retirer l'autoinjecteur du site d'injection. Ledit dispositif d'indication visuel, sonore et/ou tactile est disposé à l'extrémité arrière dudit corps 1, opposée audit site d'injection. En particulier, dans l'exemple représenté, le dispositif d'indication comporte un élément indicateur qui donne à la fois une indication visuelle, par un affichage 160 adéquat dans une ou plusieurs fenêtres 11 du corps 1, et une indication sonore, comme cela sera décrit plus en détails ci-après.

Pour éviter que l'utilisateur ne retire l'autoinjecteur du site d'injection dès la fin d'injection, l'autoinjecteur comporte un système retardateur, qui va retarder l'actionnement dudit dispositif d'indication par rapport à la fin de l'injection.

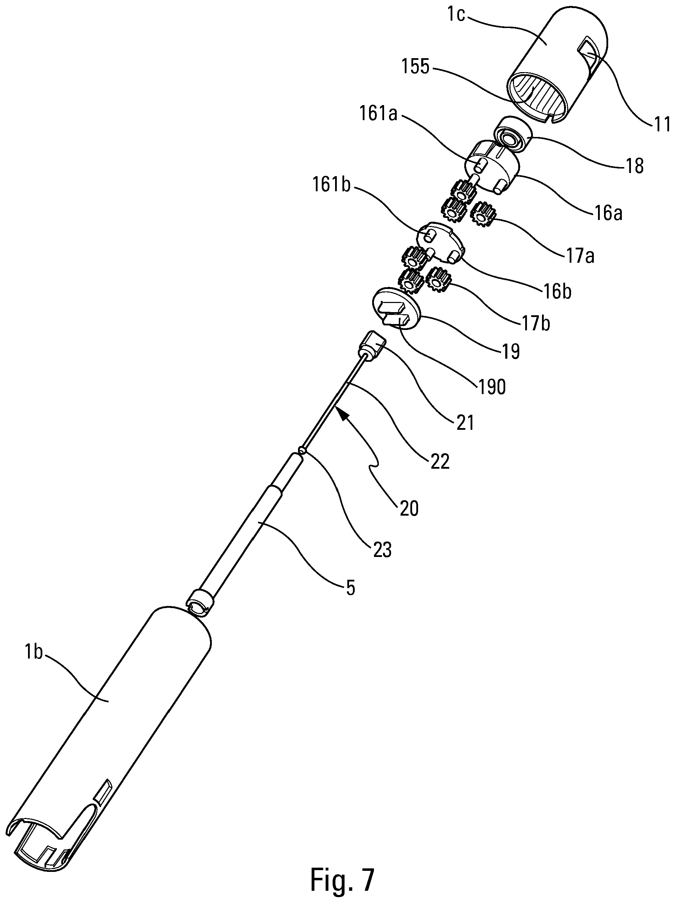

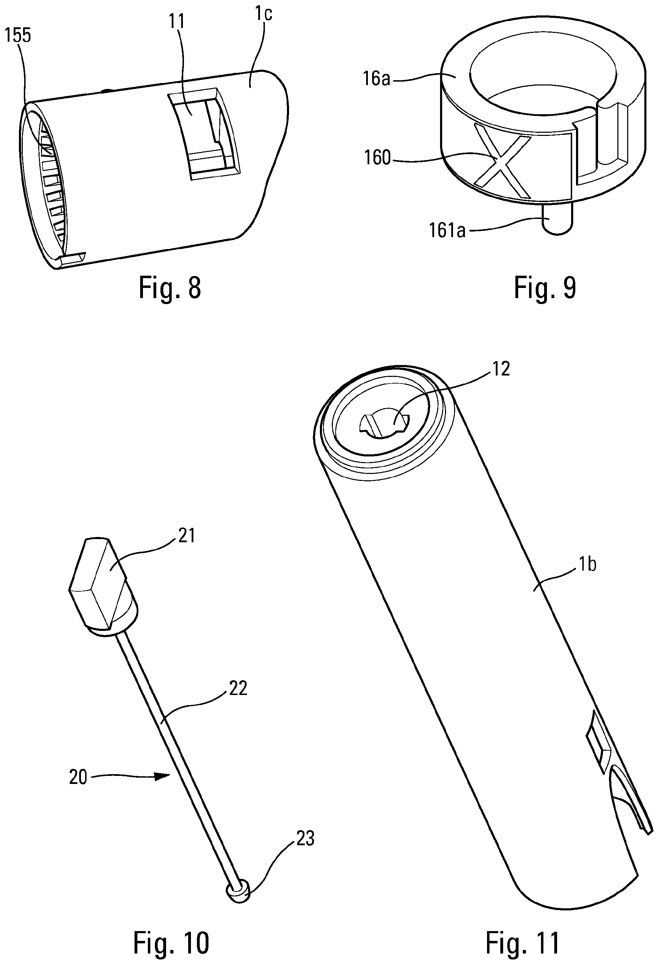

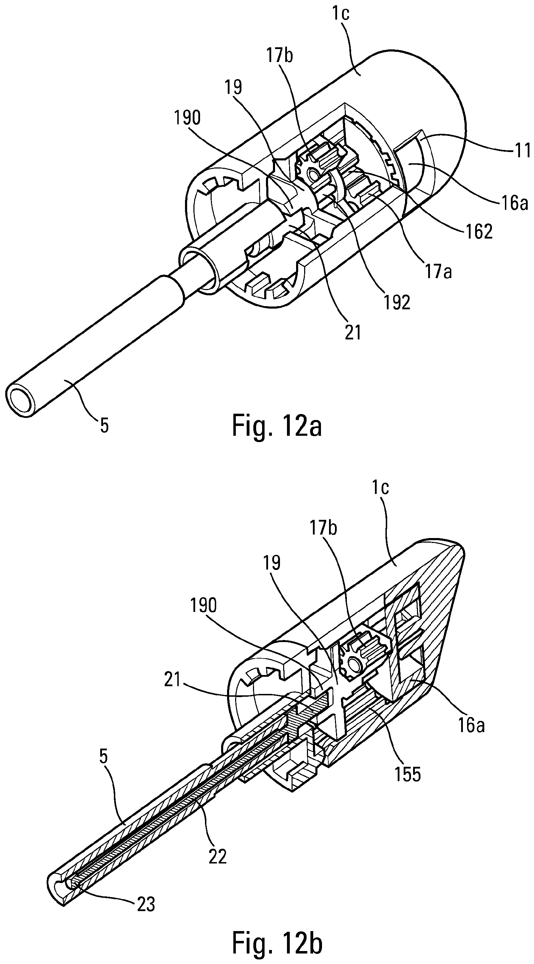

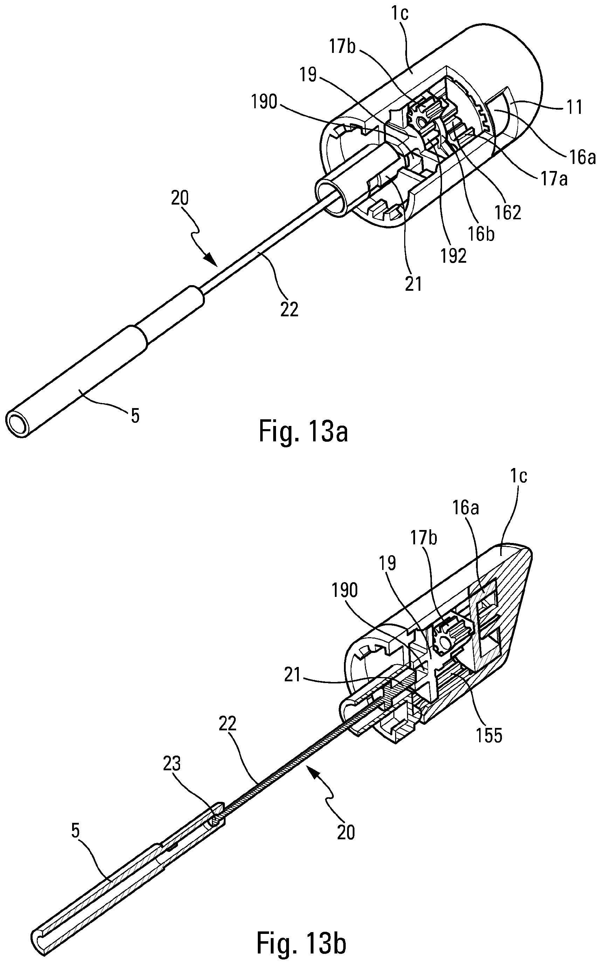

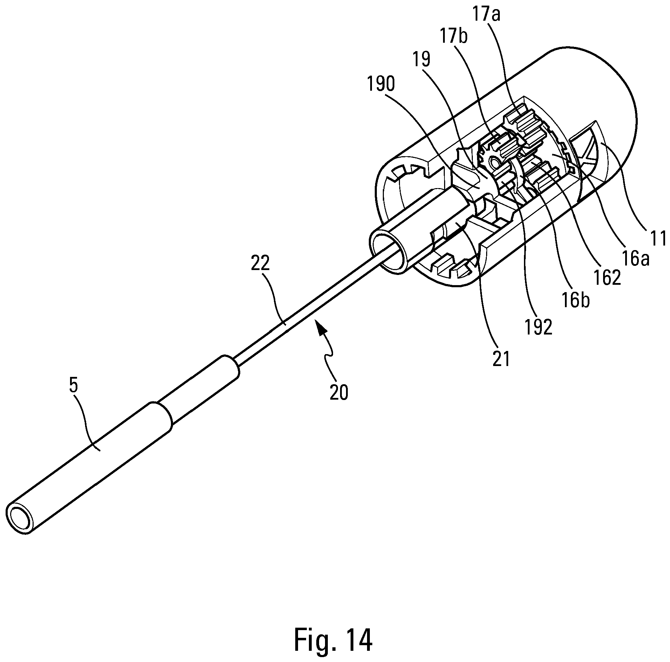

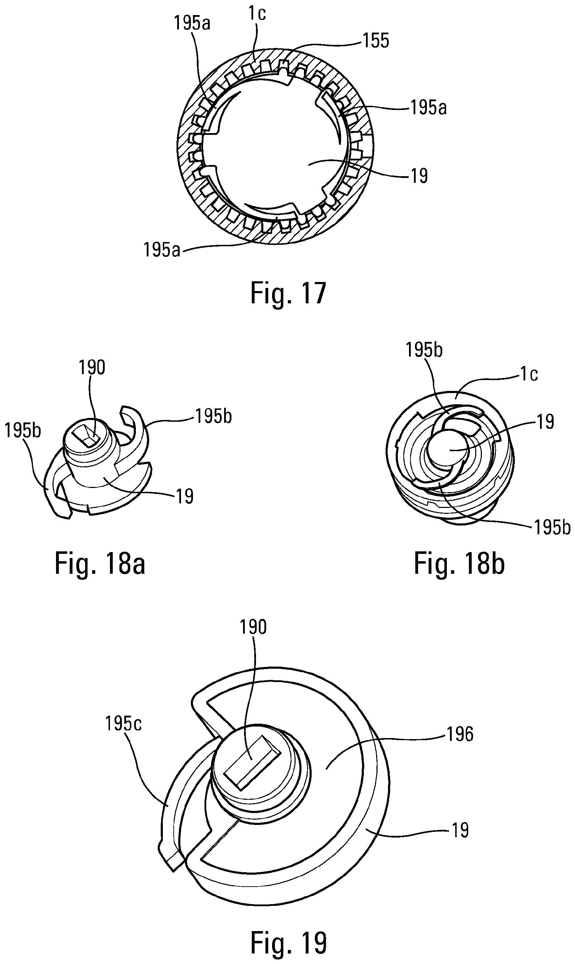

Les figures 7 à 19 illustrent un système retardateur avantageux. Les figures 7 à 16d illustrent un mode de réalisation avantageux de la présente invention, et les figures 17 à 19 illustrent des variantes avantageuses.

Ce système retardateur a principalement pour but de décaler dans le temps l'indication visuelle, sonore et/ou tactile après la fin de l'injection du produit fluide à l'intérieur dudit corps. Ceci permet notamment une diffusion pendant quelques secondes du produit après son injection. Un tel retardateur procure aussi un bénéfice pour l'utilisateur, qui n'a plus à compter, par exemple jusqu'à 10, après son injection, le temps pris pour ce comptage pouvant être très variable d'un utilisateur à un autre. Avec un retardateur, on facilite la séquence d'utilisation d'un autoinjecteur.

Le retardateur mécanique représenté sur les figures 7 à 19 permet de décaler ainsi de quelques secondes cette indication de fin d'utilisation par rapport à la fin de l'injection, ce retard étant prédéterminable.

La figure 7 illustre une vue schématique en perspective éclatée de ce système retardateur selon un mode de réalisation avantageux. Celui-ci comprend le corps supérieur 1c, un ressort retardateur 18, de préférence réalisé sous la forme d'un ressort à spirale, au moins un planétaire 16a, 16b, chacun avec au moins un satellite 17, un déclencheur 19, une clé de verrouillage 20, la tige de piston 5 et le corps intermédiaire 1b.

Chaque planétaire 16a, 16b associé à ses satellites 17 forme un étage du système retardateur. Dans l'exemple représenté sur les figures 7 à 19, il y a deux étages empilés axialement, avec un premier planétaire 16a et un second planétaire 16b, mais on peut prévoir un nombre quelconque d'étages, par exemple un seul étage ou plus de deux étages.

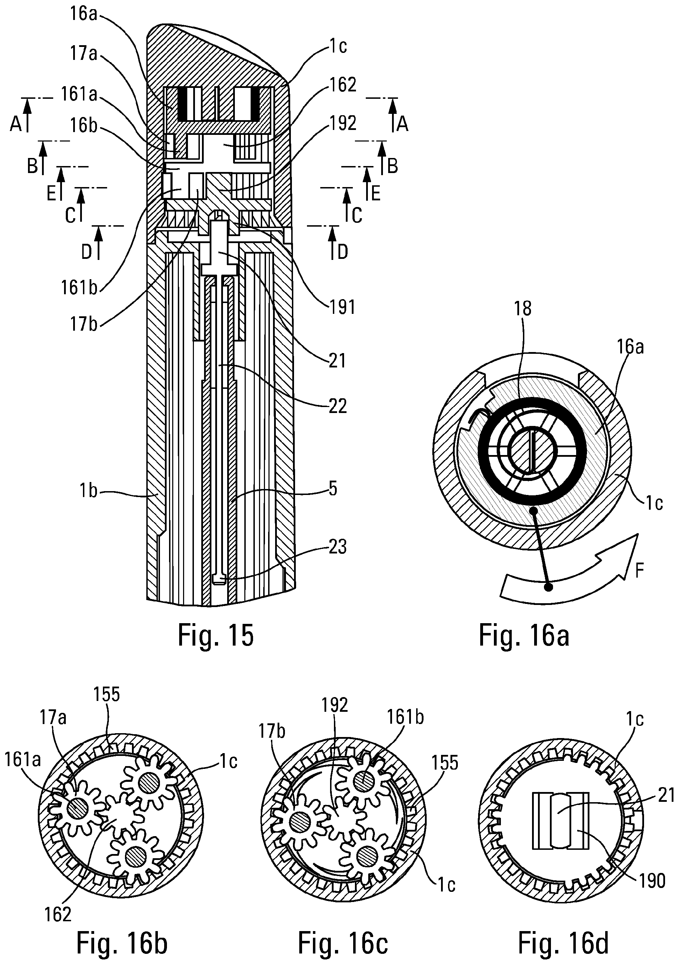

Le ressort à spirale 18 est fixé d'une part au premier planétaire 16a et d'autre part au corps supérieur 1c, comme visible dans la figure 16a. En variante, ledit ressort à spirale pourrait être fixé à un autre planétaire, par exemple le second planétaire 16b dans l'exemple représenté, ou au déclencheur 19. De plus, le ressort à spirale pourrait être fixé à une autre partie du corps 1, par exemple le corps intermédiaire 1b, ou à tout élément fixé audit corps 1.

Dans l'exemple représenté, le premier planétaire 16a forme également l'élément indicateur du dispositif d'indication visuel, sonore et/ou tactile. En variante, cet élément indicateur pourrait être formé par un autre planétaire, par exemple le second planétaire 16b dans l'exemple représenté, ou par le déclencheur.

Chaque planétaire 16a, 16b comporte une partie en forme de disque sur laquelle sont formées d'un côté une ou plusieurs tige(s) de support de satellites 161a, 161b qui reçoivent chacune de manière rotative un satellite 17a, 17b. Dans l'exemple représenté, il y a trois satellites 17a, 17b à chaque étage, de sorte qu'il y a trois tiges 161a, 161b sur chaque planétaire 16a, 16b. Un nombre quelconque de satellites est toutefois possible.

Le second planétaire 16b comporte du côté axial opposé aux tiges de support 161b, un axe central 162 pourvu d'un engrenage, et coopérant avec les satellites 17a du premier planétaire 16a.

Ainsi, comme visible sur les figures 7 et 12a à 16d, le système retardateur utilise le principe des trains épicycloïdaux. Chaque étage de ce système permet de démultiplier et/ou ralentir les rotations de l'étage précédent.

La clé de verrouillage 20 comporte une tête 21 adaptée à coopérer avec le système retardateur, une tige longitudinale 22, et en embout 23 adapté à coopérer avec la tige de piston 5.

En position avant déclenchement du système retardateur, la tête 21 de la clé de verrouillage 20 est en position de blocage dans laquelle elle coopère avec un profil correspondant 12 du corps intermédiaire 1b et avec un profil correspondant 190 du déclencheur 19, de sorte que celui-ci est bloqué en rotation par ladite clé. Lorsque la tige de piston 5 arrive vers sa position de fin d'injection, elle va coopérer avec l'embout 23 de la clé de verrouillage 20, et ainsi tirer ladite clé axialement vers le bas. De ce fait, la tête 21 de ladite clé de verrouillage 20 sort axialement dudit profil 190 du déclencheur 19, de sorte que ledit déclencheur 19 n'est plus bloqué en rotation par ladite clé 20.

Du côté axial opposé audit profil 190, le déclencheur comporte un axe central 192 pourvu d'un engrenage, et coopérant avec les satellites 17b du second planétaire 16b.

S'il n'y avait qu'un seul étage au lieu des deux représentés, il n'y aurait pas le second planétaire 16b, et ce seraient les satellites 17a du premier planétaire 16a qui coopéreraient directement avec l'axe central 192 du déclencheur 19. De même, s'il y avait plus de deux étages, il aurait au moins un planétaire supplémentaire entre le second planétaire 16b et le déclencheur 19.

Le corps supérieur 1c comporte un engrenage 155 sur sa surface interne latérale, comme clairement visible sur les figures 16b et 16c. Cet engrenage interne 155 du corps supérieur 1c coopère avec les satellites 17a,b qui sont assemblés sur les planétaires 16a,b.

Le ressort à spirale 18 sollicite le premier planétaire en rotation dans le sens de la flèche F sur la figure 16a. Cette rotation est transmise via les satellites 17a à l'axe central 162 du second planétaire 16b, et de là, via les satellites 17b à l'axe central 192 du déclencheur 19. Tant que le déclencheur 19 est bloqué par ladite clé de verrouillage 20, le système de retardement est donc également bloqué.

Lorsque le système retardateur est déclenché, le ressort 18 sollicite en rotation le premier planétaire 16a. Les satellites 17a sont donc entrainés en rotation du fait qu'il sont engrenés avec l'engrenage 155 du corps supérieur 1c. Cette rotation des satellites 17a fait donc tourner l'axe central 162 du second planétaire 16b, et la même opération se répète avec ledit second planétaire 16b. La vitesse de rotation du premier planétaire 16a est donc inférieure à celle dudit second planétaire 16b. Chaque étage supplémentaire du train épicycloïdal formant le retardateur va davantage démultiplier ces rotations, et donc davantage ralentir la rotation du premier planétaire 16a. Ainsi, avec deux étages comme représenté sur les figures, on peut ramener à un seul tour la rotation du premier planétaire 16a alors que le déclencheur 19 réalisera simultanément environ vingt tours.

Selon le nombre d'étages et/ou selon de nombre de satellites et/ou selon la forme des planétaires et/ou selon les dimensions des engrenages en jeu, on peut régler assez précisément le temps entre le moment où le système retardateur est déclenché, en fin d'injection, et le moment où le premier planétaire 16a aura réalisé sa rotation prédéfinie pour réaliser l'indication, et notamment indiquer dans la fenêtre de l'indicateur que l'autoinjecteur peut être retiré du site d'injection. L'actionnement du dispositif d'indication visuel, sonore et/ou tactile est donc retardée par rapport à la fin de l'injection, ce qui permet au produit injecté de se diffuser dans le site d'injection pendant cette durée de retard.

Le rapport des vitesses peut varier grandement, c'est-à-dire que le système peut être utilisé pour ralentir le premier planétaire 16a (rapport des vitesses supérieur à 1) ou pour ralentir le déclencheur 19 (rapport des vitesses inférieur à 1), par exemple si c'est le déclencheur qui forme l'élément indicateur du dispositif d'indication visuelle, sonore et/ou tactile.

En variantes aux satellites à engrenages coopérant avec l'engrenage 155 du corps supérieur 1c, on peut aussi envisager une transmission différente, par exemple par friction.

Un freinage par frottements peut aussi être prévu, par exemple entre le déclencheur 19 et l'engrenage interne 155 du corps supérieur 1c. La figure 17 illustre un déclencheur 19 comportant trois pattes flexibles 195a qui glissent sur l'engrenage 155 du corps supérieur 1c, pour freiner la rotation dudit déclencheur 19.

Les figures 18a et 18b illustrent une autre variante, dans laquelle le déclencheur 19 comporte deux pattes flexibles 195b, avec les extrémités de ces pattes générant un bruit pendant toute la rotation dudit déclencheur 19, par exemple sur un profil approprié du corps supérieur, et fournissant ainsi une indication sonore du fonctionnement du système retardateur: lorsque le bruit s'arrête, l'actionnement du système retardateur est terminé, et l'utilisateur peut retirer l'autoinjecteur du site d'injection. Bien entendu, ces pattes 195b peuvent simultanément aussi fournir un freinage de la rotation.

La figure 19 montre encore une autre variante, dans laquelle le déclencheur 19 comporte une patte flexible 195c, qui peut générer du bruit pendant la rotation et/ou freiner cette rotation. Dans cette variante, le déclencheur comporte une masse d'inertie 196, qui favorise le freinage.

Il est entendu que les variantes décrites ci-dessus en référence aux figures 17 à 19 pourraient aussi s'appliquer à un planétaire 16a,b plutôt qu'au déclencheur 19.

Le dispositif retardateur permet donc de décaler d'un temps prédéterminé le moment où l'indicateur va indiquer la fin d'utilisation, à partir du moment où la phase d'injection est terminée.

Une phase complète d'actionnement de l'autoinjecteur va être décrite ci-après.

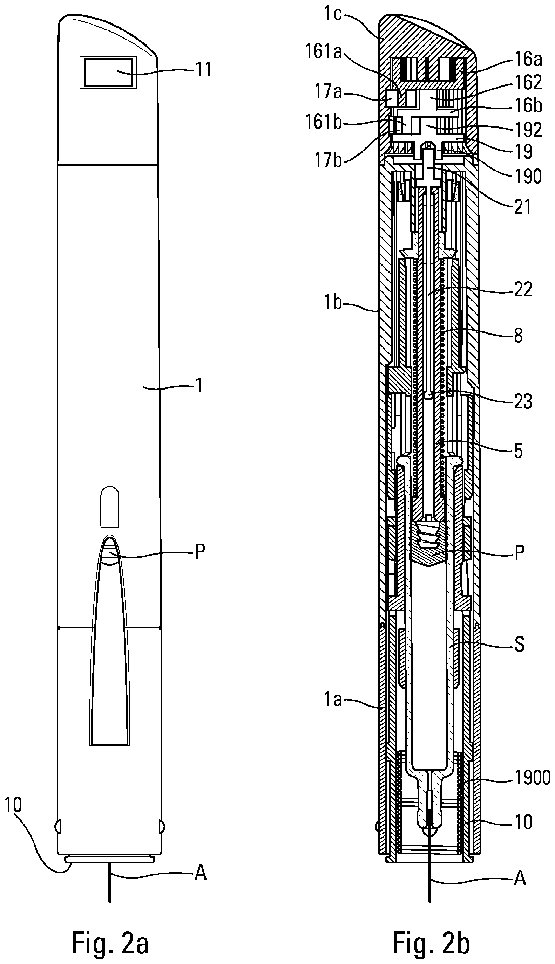

Lorsque l'utilisateur veut utiliser l'autoinjecteur, il prend le dispositif, par exemple au niveau du corps 1 et il appuie le manchon actionneur 10, qui au repos, dans sa première position projetée, fait saillie hors du corps inférieur 1, contre la partie du corps où il veut réaliser l'injection. Sur les figures 1a, 1b et 2a, 2b, on voit que la pression exercée par l'utilisateur sur le manchon actionneur 10 provoque le coulissement de celui-ci vers l'intérieur du corps 1, ce qui découvre l'aiguille et donc son piquage en raison de la pression exercée par l'utilisateur sur l'autoinjecteur.

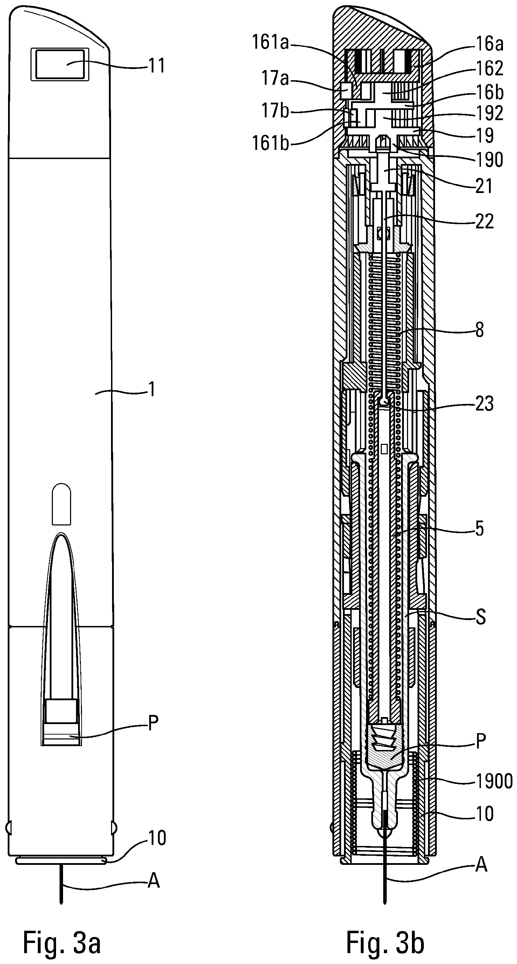

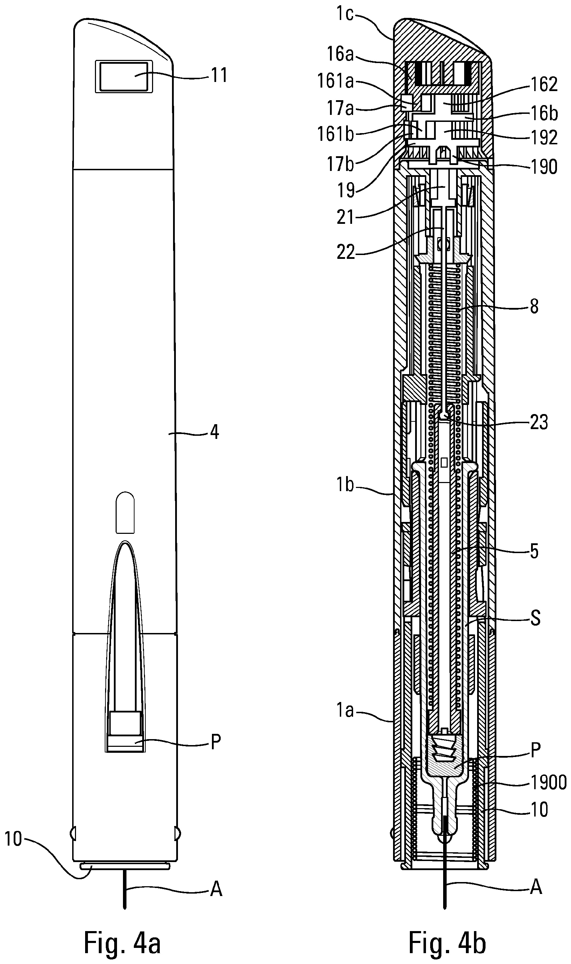

Lorsque le manchon actionneur 10 atteint sa position d'actionnement, qui est sa position d'extrémité à l'intérieur du corps 1, il provoque le déclenchement de la phase d'injection, ce qui est représenté sur les figures 3a, 3b et 4a, 4b. On constate que la tige de piston 5 coulisse à l'intérieur de la seringue A en poussant le piston P de celle-ci sous l'effet du ressort d'injection 8. Le produit est donc distribué.

A la fin de l'injection, le système retardateur est déclenché, de sorte que le dispositif d'indication ne sera actionné qu'après un temps de retard prédéterminé.

Après indication de la fin d'utilisation, lorsque l'utilisateur retire l'autoinjecteur du site d'injection, le manchon actionneur 10 est à nouveau déplacé hors du corps 1 vers la position de fin d'utilisation, qui est sa seconde position projetée, sous l'effet du ressort du manchon actionneur, avec un verrouillage dudit manchon actionneur 10, ce qui garantit une sécurité absolue pour l'utilisateur et évite tout risque de blessures avec l'aiguille après utilisation du dispositif.

Il est à noter que les première et seconde positions projetées du manchon actionneur, qui, dans l'exemple représenté, sont des positions différentes, pourraient éventuellement être identiques.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à un mode de réalisation avantageux, il est entendu que celui-ci n'est pas limitatif. En particulier, le manchon actionneur et/ou la serrure d'injection et/ou le dispositif retardateur et/ou le dispositif d'indication sonore et/ou tactile pourrai(en)t être utilisé(s) réalisés différemment. Le piquage de l'aiguille et/ou une rétractation de l'aiguille après injection pourrait être commandé par un ou plusieurs bouton(s). D'autres modifications sont également possibles pour l'homme du métier sans sortir du cadre de la présente invention tel que défini par les revendications annexées.

## Revendications

1. Autoinjecteur comportant:
- un corps (1) recevant un réservoir (S), ledit réservoir (S) contenant du produit fluide et comportant un piston (P), tel qu'une seringue pré-remplie, ledit réservoir (S) étant fixe dans ledit corps (1),
- un manchon actionneur (10) comportant une extrémité de contact (101) destinée à venir en contact avec le corps de l'utilisateur, ledit manchon actionneur (10) s'étendant au moins partiellement à l'intérieur dudit corps (1) et étant déplaçable par rapport audit corps (1) entre des positions projetées, dans lesquelles ledit manchon actionneur (10) fait au moins partiellement saillie hors dudit corps (1), et une position d'actionnement, dans laquelle ledit manchon actionneur (10) est axialement déplacé vers l'intérieur dudit corps (1), ledit manchon actionneur (10) étant dans une première position projetée avant actionnement de l'autoinjecteur et dans une seconde position projetée après actionnement de l'autoinjecteur,
- une tige de piston (5) adaptée à coopérer avec le piston (P) dudit réservoir (S), ladite tige de piston (5) étant déplaçable entre une position chargée et une position d'injection dans laquelle ladite tige de piston (5) a déplacé le piston (P) du réservoir (S) pour injecter le produit fluide dans un site d'injection,
- un dispositif d'indication visuel, sonore et/ou tactile pour indiquer à l'utilisateur qu'il peut retirer ledit autoinjecteur dudit site d'injection, ledit dispositif d'indication visuel, sonore et/ou tactile étant disposé à l'extrémité arrière dudit corps (1), opposée audit site d'injection,
**caractérisé en ce que** ledit autoinjecteur comporte un dispositif retardateur pour retarder l'actionnement dudit dispositif d'indication visuel, sonore et/ou tactile par rapport à la fin de l'injection, ledit dispositif retardateur comportant un train épicycloïdal ayant au moins un, avantageusement deux, étage(s), ledit train épicycloïdal comportant un ressort retardateur (18), au moins un planétaire (16a, 16b), chacun comportant au moins un satellite (17a, 17b), un déclencheur (19), et une clé de verrouillage (20) pour bloquer ledit déclencheur (19) en rotation jusqu'en fin d'injection, ledit ressort retardateur (18) étant réalisé sous la forme d'un ressort à spirale fixé d'une part à un planétaire (16a), formant indicateur dudit dispositif d'indication visuel, sonore et/ou tactile, et d'autre part au corps (1), ladite clé de verrouillage (20) comporte une tête (21), une tige longitudinale (22) et en embout (23) adapté à coopérer avec la tige de piston (5), dans lequel, avant déclenchement du système retardateur, la tête (21) de la clé de verrouillage (20) est en position de blocage dans laquelle elle coopère avec un profil correspondant (12) du corps (1) et avec un profil correspondant (190) du déclencheur (19), de sorte que ledit déclencheur (19) est bloqué en rotation par rapport audit corps (1) par ladite clé de verrouillage (20), dans lequel lorsque la tige de piston (5) arrive vers sa position de fin d'injection, elle coopère avec l'embout (23) de la clé de verrouillage (20) pour tirer ladite clé de verrouillage (20) axialement vers le bas hors de sa position de blocage, de sorte que ledit déclencheur (19) n'est alors plus bloqué en rotation par ladite clé de verrouillage (20).

2. Autoinjecteur selon la revendication 1, dans lequel chaque satellite (17a, 17b) d'un planétaire (16a, 16b) coopère d'une part avec ledit corps (1) et d'autre part soit avec un autre planétaire (16b, 16a), soit avec ledit déclencheur (19).

3. Autoinjecteur selon la revendication 2, dans lequel ledit corps (1) comporte un engrenage (155) sur sa surface interne latérale, ledit engrenage (155) coopérant avec au moins un satellite (17a, 17b).

4. Autoinjecteur selon la revendication 2 ou 3, dans lequel ledit déclencheur (19) comporte un axe central (192) pourvu d'un engrenage coopérant avec au moins un satellite (17a, 17b).

5. Autoinjecteur selon l'une quelconque des revendications précédentes, dans lequel ledit train épicycloïdal comporte deux planétaires (16a, 16b), chacun comportant trois satellites (17).

6. Autoinjecteur selon l'une quelconque des revendications précédentes, dans lequel ledit déclencheur (19) comporte au moins une patte flexible (195a; 195b; 195c) adaptée à coopérer avec ledit corps (1) pour générer un bruit et/ou pour freiner la rotation dudit déclencheur (19).

7. Autoinjecteur selon l'une quelconque des revendications précédentes, dans lequel ledit réservoir (S) comporte une aiguille (A) à travers laquelle ledit produit fluide est injecté dans ledit site d'injection.

## Patentansprüche

1. Autoinjektor, folgendes umfassend:
- einen Körper (1), in dem ein Vorratsbehälter (S) aufgenommen ist, wobei der Vorratsbehälter (S) ein Fluidprodukt enthält und einen Kolben (P), beispielsweise eine vorgefüllte Spritze, umfasst, wobei der Vorratsbehälter (S) in dem Körper (1) befestigt ist,
- eine Betätigungshülse (10), die ein Kontaktende (101) aufweist, das dazu bestimmt ist, mit dem Körper des Benutzers in Kontakt zu kommen, wobei sich die Betätigungshülse (10) zumindest teilweise im Inneren des Körpers (1) erstreckt und relativ zu dem Körper (1) beweglich ist zwischen vorstehenden Stellungen, in denen die Betätigungshülse (10) zumindest teilweise aus dem Körper (1) herausragt, und einer Betätigungsstellung, in der die Betätigungshülse (10) axial in den Körper (1) hinein verschoben ist, wobei sich die Betätigungshülse (10) vor der Betätigung des Autoinjektors in einer ersten vorstehenden Stellung und nach der Betätigung des Autoinjektors in einer zweiten vorstehenden Stellung befindet,
- eine Kolbenstange (5), die geeignet ist, mit dem Kolben (P) des Vorratsbehälters (S) zusammenzuwirken, wobei die Kolbenstange (5) bewegbar ist zwischen einer geladenen Stellung und einer Injektionsstellung, in der die Kolbenstange (5) den Kolben (P) des Vorratsbehälters (S) verschoben hat, um das Fluidprodukt in eine Injektionsstelle zu injizieren,
- eine Vorrichtung zur optischen, akustischen und/oder tastbaren Anzeige, mit der dem Benutzer angezeigt wird, dass er den Autoinjektor von der Injektionsstelle entfernen kann, wobei die Vorrichtung zur optischen, akustischen und/oder tastbaren Anzeige am hinteren Ende des Körpers (1), gegenüber der Injektionsstelle, angeordnet ist,
**dadurch gekennzeichnet, dass** der Autoinjektor eine Verzögerungseinrichtung zum Verzögern der Betätigung der Vorrichtung zur optischen, akustischen und/oder tastbaren Anzeige in Bezug auf das Ende der Injektion umfasst, wobei die Verzögerungseinrichtung ein Planetengetriebe mit mindestens einer, vorzugsweise zwei, Stufe(n) umfasst, wobei das Planetengetriebe eine Verzögerungsfeder (18), mindestens ein Zentralrad (16a, 16b) mit jeweils mindestens einem Planetenrad (17a, 17b), einen Auslöser (19) und einen Sperrschlüssel (20) zur Verdrehsicherung des Auslösers (19) bis zum Ende der Injektion umfasst, wobei die Verzögerungsfeder (18) in Form einer Spiralfeder ausgeführt ist, die einerseits an einem Zentralrad (16a) befestigt ist, wobei sie ein Anzeigeelement der Vorrichtung zur optischen, akustischen und/oder tastbaren Anzeige bildet, und andererseits an dem Körper (1) befestigt ist, wobei der Verriegelungsschlüssel (20) einen Kopf (21), eine Längsstange (22) und eine Spitze (23) aufweist, die geeignet ist, mit der Kolbenstange (5) zusammenzuwirken, wobei sich vor dem Auslösen des Verzögerungssystems der Kopf (21) des Sperrschlüssels (20) in einer Sperrstellung befindet, in der er mit einem entsprechenden Profil (12) des Körpers (1) und mit einem entsprechenden Profil (190) des Auslösers (19) zusammenwirkt, sodass der Auslöser (19) durch den Sperrschlüssel (20) in Bezug auf den Körper (1) verdrehgesichert ist, wobei die Kolbenstange (5) beim Erreichen ihrer Endstellung für die Injektion mit der Spitze (23) des Sperrschlüssels (20) zusammenwirkt, um den Sperrschlüssel (20) axial nach unten aus seiner Sperrstellung herauszuziehen, sodass der Auslöser (19) dann nicht mehr durch den Sperrschlüssel (20) verdrehgesichert ist.

2. Autoinjektor nach Anspruch 1, bei dem jedes Planetenrad (17a, 17b) eines Zentralrades (16a, 16b) einerseits mit dem Körper (1) und andererseits entweder mit einem anderen Zentralrad (16b, 16a) oder mit dem Auslöser (19) zusammenwirkt.

3. Autoinjektor nach Anspruch 2, bei dem der Körper an seiner seitlichen Innenfläche ein Zahnrad (155) aufweist, das mit mindestens einem Planetenrad (17a, 17b) zusammenwirkt.

4. Autoinjektor nach Anspruch 2 oder 3, bei dem der Auslöser (19) eine Mittelachse (192) umfasst, die mit einem Zahnrad versehen ist, das mit mindestens einem Planetenrad (17a, 17b) zusammenwirkt.

5. Autoinjektor nach einem der vorhergehenden Ansprüche, bei dem das Planetengetriebe zwei Zentralräder (16a, 16b) umfasst, die ihrerseits jeweils drei Satelliten (17) umfassen.

6. Autoinjektor nach einem der vorhergehenden Ansprüche, bei dem der Auslöser (19) mindestens eine flexible Lasche (195a; 195b; 195c) aufweist, die geeignet ist, mit dem Körper (1) zusammenzuwirken, um ein Geräusch zu erzeugen und/oder die Drehung des Auslösers (19) zu abzubremsen.

7. Autoinjektor nach einem der vorhergehenden Ansprüche, bei dem der Vorratsbehälter (S) eine Nadel (A) umfasst, mit der das Fluidprodukt in die Injektionsstelle injiziert wird.

## Claims

1. An autoinjector comprising:
. a body (1) receiving a reservoir (S), said reservoir (S) containing fluid and including a piston (P), such as a pre-filled syringe, said reservoir (S) being stationary in said body (1),
. an actuator sleeve (10) that includes a contact end (101) for coming into contact with the user's body, said actuator sleeve (10) extending inside said body (1) at least in part and being movable relative to said body (1) between projecting positions, in which said actuator sleeve (10) projects out from said body (1) at least in part, and an actuated position, in which said actuator sleeve (10) is moved axially into said body (1), said actuator sleeve (10) being in a first projecting position before actuation of the autoinjector, and in a second projecting position after actuation of the autoinjector,
. a piston rod (5) that is adapted to co-operate with the piston (P) of said reservoir (S), said piston rod (5) being movable between a primed position and an injection position in which said piston rod (5) has moved the piston (P) of the reservoir (S) so as to inject the fluid into an injection site,
. a visual, audible, and/or tactile indicator device for indicating to the user that said autoinjector may be removed from said injection site, said visual, audible and/or tactile indicator device being arranged at the rear end of said body (1), remote from said injection site,
said autoinjector being **characterized in that** it includes a retarding device so as to delay actuating said visual, audible, and/or tactile indicator device relative to the end of injection, said retarding device comprising an epicyclic gear train having at least one stage, advantageously two stages, said epicyclic gear train comprising a retarding spring (18), at least one planet carrier (16a, 16b), each carrying at least one planet gear (17a, 17b), a trigger (19), and a locking key (20) so as to prevent said trigger (19) from turning until the end of injection, said retarding spring (18) being made in the form of a spiral spring that is fastened firstly to a planet carrier (16a) that forms an indicator of said visual, audible and/or tactile indicator device, and secondly to the body (1), said locking key (20) comprising a head (21), a longitudinal rod (22), and an endpiece (23) that is adapted to co-operate with the piston rod (5), wherein, prior to triggering the retarding system, the head (21) of the locking key (20) is in its blocking position in which it co-operates with a corresponding profile (12) of the body (1) and with a corresponding profile (190) of the trigger (19), such that said trigger (19) is prevented from turning relative to said body (1) by said locking key (20), wherein when the piston rod (5) arrives towards its end-of-injection position, it co-operates with the endpiece (23) of the locking key (20) so as to pull said locking key (20) axially downwards out from its blocking position, such that said trigger (19) is thus no longer prevented from turning by said locking key (20).

2. An autoinjector according to claim 1, wherein each planet gear (17a, 17b) of a planet carrier (16a, 16b) co-operates firstly with said body (1) and secondly either with another planet carrier (16b, 16a) or with said trigger (19).

3. An autoinjector according to claim 2, wherein said body (1) includes a ring gear (155) on its inside surface, said ring gear (155) co-operating with at least one planet gear (17a, 17b).

4. An autoinjector according to claim 2 or claim 3, wherein said trigger (19) includes a central pin (192) that is provided with a sun gear that co-operates with at least one planet gear (17a, 17b).

5. An autoinjector according to any preceding claim, wherein said epicyclic gear train comprises two planet carriers (16a, 16b), each carrying three planet gears (17).

6. An autoinjector according to any preceding claim, wherein said trigger (19) includes at least one flexible tab (195a; 195b; 195c) that is adapted to co-operate with said body (1) so as to generate a noise and/or so as to brake the turning of said trigger (19).

7. An autoinjector according to any preceding claim, wherein said reservoir (S) includes a needle (A) through which said fluid is injected into said injection site.
